# EUROPEAN PATENT APPLICATION

(11) **EP 4 372 082 A1**
(43) Date of publication of application: **22.05.2024**
(21) Application number: 22853457.4
(22) Date of filing: 03.08.2022
(51) Int. Cl.: C12N 9/24, C12N 15/52, C12P 19/02

(54) **VARIANT POLYPEPTIDE HAVING XYLANASE ACTIVITY**

(30) Priority: 06.08.2021 KR 20210103849
(71) Applicant: CJ CheilJedang Corporation, Seoul 04560 (KR)
(72) Inventor: LEE, Surin, Seoul 04560 (KR); SON, Byung-sam, Seoul 04560 (KR); SHIM, Jihyun, Seoul 04560 (KR); CHOI, Eun Jung, Seoul 04560 (KR)
(74) Representative: Michalski Hüttermann & Partner Patentanwälte mbB
(86) International application number: PCT/KR2022/011477
(87) International publication number: WO 2023/014081

(57) **Abstract**

The present disclosure relates to a modified polypeptide having xylanase activity and use thereof.

## Description

### [Technical Field]

The present disclosure relates to a modified polypeptide having xylanase activity and use thereof.

### [Background Art]

Xylanase (EC 3.2.1.8) is a hydrolase that randomly degrades the β-1,4 backbone of xylan, which is a component of plant cell walls. Xylanase is mainly used to break down biomass in fields such as animal feeds, baking, pulp bleaching, etc. (Beg QK, Kapoor M, Mahajan L, Hoondal GS. Microbial xylanases and their industrial applications: a review. Appl Microbiol Biotechnol. 2001 Aug;56(3-4):326-38. doi: 10.1007/s002530100704. PMID: 11548999.). However, in some of these fields, resistance and activity under harsh conditions (high temperature, basic conditions) are required.

### [Disclosure]

### [Technical Problem]

For the convenient use of xylanase employed in various fields, its resistance and activity under harsh conditions (high temperature, basic conditions) are required, but most xylanases have low thermal stability, and therefore, it is difficult to apply xylanase in various fields.

### [Technical Solution]

An object of the present disclosure is to provide a modified polypeptide having xylanase activity.

Another object of the present disclosure is to provide a composition including the modified polypeptide.

Still another object of the present disclosure is to provide use of the modified polypeptide or the composition for the reaction with a xylan-containing material.

Still another object of the present disclosure is to provide a method of degrading xylan-containing materials; and/or a method of producing xylo-oligosaccharides or xylose, the method including: bringing the modified polypeptide, a host cell expressing the modified polypeptide, and/or a composition including the modified polypeptide into contact with a xylan-containing material.

Still another object of the present disclosure is to provide a polynucleotide encoding the modified polypeptide; a nucleic acid construct including the polynucleotide; a vector including the polynucleotide or the nucleic acid construct; and/or a host cell including the polynucleotide, the nucleic acid construct, or the vector.

Still another object of the present disclosure is to provide a method of preparing the modified polypeptide.

### [Advantageous Effects]

A modified polypeptide having xylanase activity of the present disclosure may be usefully applied to a variety of industrial fields.

### [Brief Description of the Drawing]

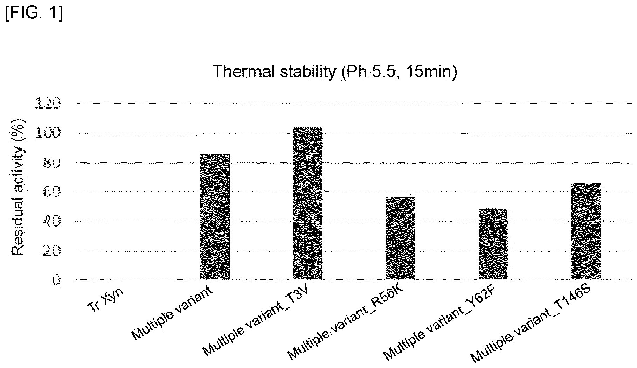

FIG. 1 shows the results of confirming thermal stability of a modified polypeptide of the present disclosure.

### [Detailed Description of Preferred Embodiments]

An aspect of the present disclosure provides a modified polypeptide having xylanase activity.

In one embodiment, i) the modified polypeptide is a polypeptide having a sequence identity of 70% or more and less than 100% with SEQ ID NO: 1; and/or
ii) the modified polypeptide is a polypeptide encoded by a polynucleotide having a sequence identity of 70% or more and less than 100% with a sequence encoding a mature polypeptide of SEQ ID NO: 1; and/or
iii) the modified polypeptide is a polypeptide encoded by (a) a mature polypeptide coding sequence of SEQ ID NO: 1, (b) a cDNA thereof, or (c) a polynucleotide that hybridizes to the full-length complement of (a) or (b) under low stringency conditions, medium stringency conditions, medium-high stringency conditions, high stringency conditions, or very high stringency conditions; and/or
iv) the modified polypeptide is a functional fragment of i), ii) or iii) polypeptide having xylanase activity; and
the modified polypeptide includes any one or more selected from the following modifications:
   substitutions of one or more amino acids at positions 2, 3, 28, 38, 56, 62, 65, 80, 93, 114, 144, 146, 149, 151, 161, and 165 with another amino acids;
   wherein the position number is a position corresponding to the position of the polypeptide of SEQ ID NO: 1.

In any one embodiment of the above embodiments, before modification, the amino acid at position 3 may be isoleucine (I); the amino acid at position 56 may be lysine (K); the amino acid at position 62 may be phenylalanine (F); and/or the amino acid at position 146 may be serine (S).

In any one embodiment of the above embodiments, the modified polypeptide may include modifications of amino acids at positions selected from below:
position 3;
position 56;
position 62;
position 146;
positions 3 + 56;
positions 3 + 62;
positions 3 + 146;
positions 56 + 62;
positions 56 + 146;
positions 62 + 146;
positions 3 + 56 + 62;
positions 3 + 56 + 146;
positions 3 + 62 + 146;
positions 56 + 62 + 146; and
positions 3 + 56 + 62 + 146;
wherein the position number is a position corresponding to the position of the polypeptide of SEQ ID NO: 1.

In any one embodiment of the above embodiments, the modified polypeptide may include any one or more substitutions from i) to vi) below;
i) substitution of an amino acid at position 3 with valine or threonine;
ii) substitution of an amino acid at position 56 with arginine;
iii) substitution of an amino acid at position 62 with tyrosine; and
iv) substitution of an amino acid at position 146 with threonine; wherein the position number is a position corresponding to the position of the polypeptide of SEQ ID NO: 1.

In any one embodiment of the above embodiments, the modified polypeptide may include substitutions of 15 or more of positions 2, 3, 28, 38, 56, 62, 65, 80, 93, 114, 144, 146, 149, 151, 161, and 165.

In any one embodiment of the above embodiments, the modified polypeptide may include any one or more substitutions selected from below:
I3T/V ;
K56R ;
F62Y ;
S146T ;
I3T/V + K56R ;
I3T/V + F62Y ;
I3T/V + S146T;
K56R + F62Y ;
K56R + S146T;
F62Y + S146T;
I3T/V + K56R + F62Y ;
I3T/V + K56R + S146T ;
I3T/V + F62Y + S146T;
K56R + F62Y + S146T;
I3T/V + K56R + F62Y + S146T ;
I3T/V + T2C + T28C + N38D + S65T + S80T + F93Y + V114T + H144K + S149T + N151T + Q161R + T165V;
K56R + T2C + T28C + N38D + S65T + S80T + F93Y + V114T + H144K + S149T + N151T + Q161R + T165V;
F62Y + T2C + T28C + N38D + S65T + S80T + F93Y + V114T + H144K + S149T + N151T + Q161R + T165V;
S146T + T2C + T28C + N38D + S65T + S80T + F93Y + V114T + H144K + S149T + N151T + Q161R + T165V;
I3T/V + K56R + T2C + T28C + N38D + S65T + S80T + F93Y + V114T + H144K + S149T + N151T + Q161R + T165V;
I3T/V + F62Y + T2C + T28C + N38D + S65T + S80T + F93Y + V114T + H144K + S149T + N151T + Q161R + T165V;
I3T/V + S146T + T2C + T28C + N38D + S65T + S80T + F93Y + V114T + H144K + S149T + N151T + Q161R + T165V;
K56R + F62Y + T2C + T28C + N38D + S65T + S80T + F93Y + V114T + H144K + S149T + N151T + Q161R + T165V;
K56R + S146T + T2C + T28C + N38D + S65T + S80T + F93Y + V114T + H144K + S149T + N151T + Q161R + T165V;
F62Y + S146T + T2C + T28C + N38D + S65T + S80T + F93Y + V114T + H144K + S149T + N151T + Q161R + T165V;
I3T/V + K56R + F62Y + T2C + T28C + N38D + S65T + S80T + F93Y + V114T + H144K + S149T + N151T + Q161R + T165V;
I3T/V + K56R + S146T + T2C + T28C + N38D + S65T + S80T + F93Y + V114T + H144K + S149T + N151T + Q161R + T165V;
I3T/V + F62Y + S146T + T2C + T28C + N38D + S65T + S80T + F93Y + V114T + H144K + S149T + N151T + Q161R + T165V;
K56R + F62Y + S146T + T2C + T28C + N38D + S65T + S80T + F93Y + V114T + H144K + S149T + N151T + Q161R + T165V; and
I3T/V + K56R + F62Y + S146T + T2C + T28C + N38D + S65T + S80T + F93Y + V114T + H144K + S149T + N151T + Q161R + T165V.

In any one embodiment of the above embodiments, the modified polypeptide may have increased thermal tolerance and/or thermal stability compared to a polypeptide composed of the amino acid sequence of SEQ ID NO: 1.

Another aspect of the present disclosure provides a composition including the modified polypeptide of the present disclosure.

Still another aspect of the present disclosure provides a composition for the reaction with a xylan-containing material, the composition including the modified polypeptide of the present disclosure.

Still another aspect of the present disclosure provides use of the modified polypeptide and/or the composition including the modified polypeptide for the reaction with a xylan-containing material.

Still another aspect of the present disclosure provides a method of producing xylose and/or xylo-oligosaccharide, the method including: bringing the modified polypeptide, a host cell expressing the modified polypeptide, and/or the composition including the modified polypeptide into contact with xylan.

Still another aspect of the present disclosure provides a method of producing xylose and/or xylo-oligosaccharide, the method including: bringing the modified polypeptide, a host cell expressing the modified polypeptide, and/or the composition including the modified polypeptide into contact with a xylan-containing material.

Still another aspect of the present disclosure provides a method of degrading xylan-containing materials, the method including: treating the modified polypeptide, a host cell expressing the modified polypeptide, and/or the composition including the modified polypeptide to a xylan-containing material.

Still another aspect of the present disclosure provides a polynucleotide encoding the modified polypeptide.

Still another aspect of the present disclosure provides a nucleic acid construct including the polynucleotide.

Still another aspect of the present disclosure provides a vector including the polynucleotide or the nucleic acid construct.

Still another aspect of the present disclosure provides a host cell including the modified polypeptide, the polynucleotide, the nucleic acid construct, and/or the vector.

Still another aspect of the present disclosure provides a method of preparing the modified polypeptide, the method including the steps of culturing the host cell; and recovering the modified polypeptide expressed in the culturing step.

### [Mode for Carrying Out the Invention]

Hereinafter, the present disclosure will be described in detail. Meanwhile, each description and embodiment disclosed herein may be applied to other descriptions and embodiments, respectively. That is, all combinations of various elements disclosed herein fall within the scope of the present disclosure. Further, the scope of the present disclosure is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present disclosure.

As used in the specification and appended claims, the singular forms ("a", "an", and "the") include plural referents unless the context clearly states otherwise. Unless the context indicates otherwise, singular terms shall include the plural and plural terms shall include the singular. As used in the specification and appended claims, unless stated otherwise, the use of "or" may be used to include "and/or".

As used herein, the term "about" may be presented before a particular numerical value. The term "about" used herein includes not only the exact number recited after the term, but also a range that is near or close to that number. Considering the context in which the number is presented, it may be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third..." "i), ii), iii)..." or "(a), (b), (c), (d)..." are used to distinguish similar constitutions, and these terms do not mean that the constitutions are performed continually or sequentially. For example, when the terms are used in reference to steps of a method, use, or assay, there may be no time interval between these steps, or they may be performed concurrently, or may be performed several seconds, several minutes, several hours, several days, or several months apart.

As used herein, the term "consisting essentially of" may mean that, in the case where the features of the object claimed herein are not substantially affected by the presence of an unspecified component, the unspecified component may be present.

As used herein, the term, "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising/including" means the presence of features, steps or components recited after the term, and does not exclude the presence or addition of one or more features, steps or components. The components or features recited after the term "comprising/including" herein may be essential or mandatory. However, in some embodiments, the term may further include any other or non-essential components or features.

As used herein, the term "comprising/including" may be modified to refer to "consisting essentially of" or "consisting of" in some embodiments.

With respect to amino acid sequences in the present disclosure, although it is described as a polypeptide "comprising/including" an amino acid sequence described by a specific sequence number, a polypeptide "consisting of" an amino acid sequence described by a specific sequence number, or a polypeptide or protein "having" an amino acid sequence described by a specific sequence number, it is apparent that any protein having an amino acid sequence in which part of the sequence is deleted, modified, substituted, conservative substituted or added may be used in the present disclosure if it has the same or corresponding activity as the polypeptide consisting of the amino acid sequence of the corresponding sequence number. For example, it may be a case where the N-terminus and/or C-terminus of the amino acid sequence is added with a sequence that does not alter the function of the protein, a naturally occurring mutation, a silent mutation thereof, or a conservative substitution, but is not limited thereto.

As used herein, the term "protein" or "polypeptide" refers to a polymer or oligomer of consecutive amino acid residues. In the present disclosure, the "polypeptide", "protein", and "peptide" may be used interchangeably with "amino acid sequence".

In some cases, an amino acid sequence exhibiting activity may be referred to as an "enzyme". In the present disclosure, unless indicated otherwise, amino acid sequences are described in an N-terminal to C-terminal direction.

As used herein, the term "recombinant" with respect to a cell, or nucleic acid, polypeptide, or vector, indicates that the cell, nucleic acid, polypeptide or vector has been modified by the introduction of a heterologous nucleic acid or polypeptide or the alteration of a native nucleic acid or polypeptide, or that the cell is derived from a cell thus modified. Therefore, for example, recombinant cells may express genes that are not found within the native (non-recombinant) form of the cells, or may express native genes that are otherwise abnormally expressed, under expressed or not expressed at all.

As used herein, the term "isolated" refers to a substance which exists in a non-naturally occurring environment or does not exist naturally. It includes substantial isolation of a substance (sequence, enzyme or nucleic acid) from a substance which is naturally associated and found in nature, for example, at least one other component having a sequence, enzyme or nucleic acid.

For example, an isolated sequence, enzyme or nucleic acid provided herein may be provided in a form that is substantially free of one or more contaminants.

Examples of isolated substances include i) any substance that is non-naturally occurring; ii) any substance (e.g., enzyme, variant, nucleic acid, protein, peptide or cofactor) from which one, or more, or all naturally-occurring components associated in nature have been removed; iii) any substance that has been artificially modified from a substance found in nature; or iv) any substance that has been modified to alter the amount of that substance relative to other components associated in nature (e.g., increase in copy number of a gene encoding a specific substance; modification of a promoter naturally linked to a gene encoding a specific substance into a highly active promoter, etc.), but are not limited thereto.

As used herein, the term "wild-type" refers to a naturally-occurring state without artificial modification. When the term "wild-type" is used in reference to a polypeptide, it refers to a naturally occurring polypeptide, which does not have artificial mutations (substitutions, insertions, deletions, etc.) in one or more amino acid positions. Similarly, when the term "wild-type" is used in reference to a polynucleotide, it means not having artificial modifications (substitutions, insertions, deletions) in one or more nucleotides. However, polynucleotides encoding wild-type polypeptides are not limited to wild-type polynucleotides, and include sequences encoding any wild-type polypeptides.

As used herein, a parent sequence or backbone refers to a reference sequence in which modification is introduced to create a modified polypeptide. That is, the parent sequence may be served as a starting sequence in which modification such as substitutions, additions, and/or deletions may be introduced. The parent sequence may be naturally-occurring or wild-type, or a variant in which one or more substitutions, insertions or deletions have occurred in the natural or wild type, or may be an artificially synthesized sequence. When the parent sequence is an amino acid sequence exhibiting activity, i.e., an amino acid sequence of an enzyme, it may be referred to as a parent enzyme.

As used herein, the term "reference sequence" means a sequence used to determine the position of amino acids within any amino acid sequence. Any amino acid sequence may be aligned with a reference sequence to determine the position of an amino acid that corresponds to a particular position in the reference sequence within any amino acid sequence.

With respect to amino acid or nucleic acid sequences in the present disclosure, the term "fragment" means a part of a parent sequence. For example, it may be a polypeptide in the form in which one or more amino acids are removed from the C- or N-terminus of the parent sequence.

As used herein, the term "fragment" of an enzyme may refer to a "functional fragment". The "functional fragment" may also be referred to as an active fragment, and means a polypeptide that is a part of a parent enzyme and has enzymatic activity of the parent enzyme. For example, the functional fragment of an enzyme may include a catalytic site of the enzyme.

The fragment of the enzyme may include a part of the full length of the parent enzyme. For example, the fragment of the enzyme may include amino acids of at least about 20%, 30%, 40%, 50%, 60%, 70%, 80%, 90%, 95%, 98%, 99% or more, or less than 100% of the full length of the parent enzyme, but is not limited thereto.

As used herein, the term "modifying" means changing or altering. This may be altering from a naturally occurring state. In one example, an enzyme may be altered in such a way that the enzyme is altered from a parent or reference sequence.

In the present disclosure, a modified enzyme may be an enzyme that does not exist as it is in nature, i.e., a non-naturally occurring enzyme.

As used herein, the term "modified" means being altered, e.g., from its naturally occurring form. The modified enzyme of the present disclosure includes non-naturally occurring enzymes or naturally occurring variants. For example, the modified enzyme of the present disclosure is a modified enzyme that has not been found in nature. For example, the modified enzyme of the present disclosure may be a non-spontaneously occurring enzyme, but is not limited thereto.

As used herein, the term "modification", when used with respect to amino acid/nucleic acid sequences, may include substitution of an amino acid/nucleic acid residue of a parent sequence for a different amino acid/nucleic acid residue in one or more positions in an amino acid sequence; deletion of an amino acid/nucleic acid residue (or series of amino acid/nucleic acid residues) of a parent sequence in one or more positions; insertion of an amino acid/nucleic acid residue (or series of amino acid/nucleic acid residues) of a parent sequence in one or more positions; or truncation of the N-terminal and/or C-terminal amino acid sequences or 5' and/or 3' nucleic acid sequences, and any combination thereof.

As used herein, the term "variant" or "modified polypeptide" of an enzyme refers to a protein having one or more amino acids different from the parent enzyme by conservative substitution and/or modification. The "variant" or "modified polypeptide" may be used interchangeably. The variant or modified polypeptide may be non-naturally occurring, but is not limited thereto.

The variant differs from the sequence of the parent enzyme by one or more modifications, e.g., substitutions, deletions and/or insertions of amino acids.

Such variants may be generally identified by modifying one or more amino acids from the parent enzyme and evaluating properties of the modified protein. That is, the ability of the variants may be enhanced, unchanged or reduced relative to the parent enzyme.

Further, some variants may include modified polypeptides in which one or more regions, such as an N-terminal leader sequence or transmembrane domain, have been removed.

Other variants may include those in which a part has been removed from the N- and/or C-terminal of a mature protein.

The term "variant" or "modified polypeptide" may be used interchangeably with terms such as modification, modified protein, mutant, mutein, divergent, variant, etc., and is not limited as long as the terms are used to indicate mutation.

The variants may also include deletion or addition of amino acids that have minimal influence on the properties and secondary structure of a polypeptide. For example, the polypeptides may be conjugated with a signal (or leader) sequence at the N-terminal involved in the translocation of proteins co-translationally or post-translationally. Further, the polypeptides may also be conjugated with another sequence or linker to identify, purify, or synthesize the polypeptides.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue.

Throughout the entire specification of the present disclosure, the conventional one-letter and three-letter codes for naturally occurring amino acids are used. Further, the amino acids mentioned herein are abbreviated according to the nomenclature rules of IUPAC-IUB as follows:

| | |
|---|---|
| alanineAla, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

Meanwhile, any amino acid may be described as Xaa or X.

Further, three-letter codes generally allowed not only for naturally occurring amino acids, but also for other amino acids, such as 2-Aminoisobutyric acid (Aib), Sar (N-methylglycine), α-methyl-glutamic acid, etc. may be used.

Amino acids may be generally classified based on similarity of polarity, charge, solubility, hydrophobicity, hydrophilicity, and/or amphipathic nature of a residue. Accordingly, amino acid substitution may generally occur based on similarity of polarity, charge, solubility, hydrophobicity, hydrophilicity, and/or amphipathic nature of a residue.

For example, among the amino acids having an electrically charged side chain (electrically charged amino acid), positively charged (basic) amino acids include arginine, lysine, and histidine; negatively charged (acidic) amino acids include glutamic acid and aspartic acid. Among the amino acids having an uncharged side chain (uncharged amino acid), nonpolar amino acids include glycine, alanine, valine, leucine, isoleucine, methionine, phenylalanine, tryptophan, and proline; polar or hydrophilic amino acids include serine, threonine, cysteine, tyrosine, asparagine, and glutamine; and among the nonpolar amino acids, aromatic amino acids include phenylalanine, tryptophan, and tyrosine.

As used herein, the term "gene" means a polynucleotide that encodes a polypeptide and a polynucleotide including regions the upstream and downstream of the coding region. In some embodiments, a gene may have a sequence (intron) inserted between respective coding regions (exons).

As used herein, the term "homology" or "identity" refers to a degree of relevance between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and may be used with a default gap penalty established by the program being used. Substantially, homologous or identical sequences may generally hybridize under moderately or highly stringent conditions to the full-length of the sequence or at least about 50%, 60%, 70%, 80%, or 90% or more of the full-length. It is apparent that hybridization also includes hybridization of a polynucleotide with a polynucleotide containing a general codon or a codon in consideration of codon degeneracy.

Whether any two polynucleotide or polypeptide sequences have a homology, similarity, or identity may be, for example, determined by a known computer algorithm such as the "FASTA" program using default parameters, as in Pearson et al., (1988) [Proc. Natl. Acad. Sci. USA 85]: 2444. Alternatively, it may be determined by the Needleman-Wunsch algorithm (Needleman and Wunsch, 1970, J. Mol. Biol. 48: 443-453), as performed using the Needleman program of the EMBOSS package (EMBOSS: The European Molecular Biology Open Software Suite, Rice et al., 2000, Trends Genet. 16: 276-277) (version 5.0.0 or later) (GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073). For example, the homology, similarity, or identity may be determined using BLAST or ClustalW of the National Center for Biotechnology Information (NCBI), but is not limited thereto.

The homology, similarity, or identity of polynucleotides or polypeptides may be, for example, determined by comparing sequence information using, for example, the GAP computer program, such as Needleman et al. (1970), J Mol Biol. 48: 443, as disclosed in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have a homology, similarity or identity with each other may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York), but is not limited thereto.

As used herein, the term "mature polypeptide" means a polypeptide in a form without a signal sequence or pro-peptide sequence. A mature protein/polypeptide/peptide may be a functional form of a protein/polypeptide/peptide. The mature polypeptide may be in a final form after translation or post-translational modification. Examples of the post-translational modification include N- or C-terminal modifications, glycosylation, phosphorylation, leader sequence removal etc., but are not limited thereto.

As used herein, the term "nucleic acid construct" refers to a single- or double-stranded nucleic acid molecule, which includes one or more regulatory sequences, and which is artificially synthesized, or engineered to include a specific sequence in a manner that does not exist in nature, or isolated from nature.

As used herein, the term "expression" includes any step involved in the production of a polypeptide, e.g., transcription, post-transcriptional modification, translation, post-translational modification, and secretion, etc., but is not limited thereto.

As used herein, the term "expression vector" refers to a linear or circular nucleic acid molecule including a coding sequence and an operably linked regulatory sequence for expression thereof.

As used herein, the term "operably linked" refers to a constitution of placing a regulatory sequence to an appropriate position to regulate expression of a coding sequence. Thus, the term "operably linked" includes an attachment or linking between a regulatory region of a functional domain having a known or desired activity such as a promoter, a terminator, a signal sequence, or an enhancer, and a target (gene or polypeptide) such that the expression, secretion, or function of the target may be regulated in accordance with the known or desired activity.

As used herein, the term "cDNA" refers to a DNA sequence which may be prepared by reverse transcription from a mature, spliced mRNA molecule obtained from a eukaryotic or prokaryotic cell. The cDNA sequence lacks intron sequences that may exist in the corresponding genomic DNA. The initial primary RNA transcript is a precursor to mRNA which is processed through a series of steps including splicing before appearing as mature spliced mRNA.

As used herein, the term "regulatory sequence" refers to a polynucleotide sequence necessary for the expression of a coding sequence. Each regulatory sequence may be native (derived from the same origin) or foreign (derived from different genes) to the coding sequence. Examples of the regulatory sequence may include a leader sequence, a polyadenylation sequence, a pro-peptide sequence, a promoter, a signal peptide sequence, an operator sequence, a sequence encoding a ribosome-binding domain, and a sequence regulating the termination of transcription and translation. The minimum units of the regulatory sequence may include a promoter, and a sequence for terminating transcription and translation.

In order to describe the variants provided in the present disclosure, the following nomenclature is used.

In the present disclosure, referring to a specific position in an amino acid sequence may include referring to an amino acid present or substituted at that position. Referring to an amino acid at a specific position may be described in various ways. For example, the "position 003" may be described as "position 3", "amino acid 3", "3^{rd} amino acid". Further, for example, when the amino acid at position 3 is serine (S), it may be described as "S3" or "Ser3".

Amino acid substitution may be expressed by describing the amino acid before substitution, the position, and the amino acid to be substituted in sequence. The amino acids may be expressed using the conventional one-letter and three-letter codes. In one example, when alanine, an amino acid at position 6 of a specific sequence, is substituted with valine, it may be described as "A8V" or "Ala8Val".

Any amino acid at a specific position may be referred to as "X". For example, X6 refers to any amino acid at position 6. In addition, when the amino acid to be substituted is expressed as X, it means that it is substituted with an amino acid different from the amino acid present before substitution. For example, "V6X" indicates that V at position 6 is substituted with any amino acid other than V.

Different alterations may be expressed by simultaneously describing several types of amino acids using symbols, such as "/" or ",". For example, when the amino acid (D) at position 12 is substituted with S or K, it may be described in combination of D12S/K or D12S,K. As another example, P/S197K means that the amino acid P or S at position 197 before substitution is substituted with K.

Multiple mutations may be described using "+". For example, descriptions such as "G2A+ M8V" mean that glycine, an amino acid at position 2, is substituted with alanine, and methionine, an amino acid at position 8, is substituted with valine, respectively.

Amino acid deletion may be expressed by describing the amino acid before substitution, the position, and * in sequence. In one example, when alanine, an amino acid at position 8 of a specific sequence, is deleted, it may be described as A8* or (Ala8*).

Amino acid insertion, for example, when lysine is inserted between glycine at position 8 and an amino acid at position 9 in a specific sequence, may be described as Gly8GlyLys or G8GK. When one or more amino acids are inserted, for example, when lysine and valine are inserted between glycine at position 8 and an amino acid at position 9 in a specific sequence, it may be described as Gly8GlyLysVal or G8GKV. In this case, the position of the inserted amino acid may be expressed using the amino acid number and alphabet in front of the inserted amino acid. For example, in the above case, the inserted lysine and valine may be numbered as 8aK and 8bV.

As used herein, the term "corresponding to" refers to an amino acid residue at the position recited in a protein or peptide, or an amino acid residue which is similar, identical, or homologous to the residue recited in a protein or peptide. Identifying an amino acid at a corresponding position may be determining a particular amino acid in a sequence that refers to a particular sequence. As used herein, the "corresponding region" generally refers to a similar or corresponding position in the related protein or reference protein.

In the present disclosure, SEQ ID NO: 1 may be used as a reference sequence to determine the position of amino acids in any amino acid sequence.

That is, SEQ ID NO: 1 disclosed herein may be used to determine the corresponding amino acid residues in any polypeptide having xylanase activity. Unless indicated otherwise in the present disclosure, residues of a particular amino acid sequence are numbered based on SEQ ID NO: 1.

For example, any amino acid sequence is aligned with SEQ ID NO: 1, and based on the alignment, each amino acid residue of the amino acid sequence may be numbered with reference to the numerical position of the amino acid residue corresponding to the amino acid residue of SEQ ID NO: 1. For example, a sequence alignment algorithm such as that described herein may identify the position of an amino acid or a position where modifications such as substitutions, insertions or deletions occur, compared to a query sequence (also referred to as a "reference sequence").

In such alignment, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000, Trends Genet. 16: 276-277), etc. may be used, but is not limited thereto.

Further, the corresponding amino acid residue in another xylanase may be identified by multiple sequence alignment. Examples of the multiple sequence alignment known in the art include, but are not limited to, programs such as MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22: 4673-4680) using their respective default parameters.

In addition, when enzymes diverged from the mature polypeptide of SEQ ID NO: 1 fail to detect their relationship by traditional sequence-based comparison, other pairwise sequence comparison algorithms may be used (Lindahl and Elofsson, 2000, J. Mol. Biol. 295: 613-615). Higher sensitivity in sequence-based searching may be attained using search programs that utilize probabilistic representations of polypeptide families (profiles) to search databases. For example, the PSI BLAST program generates profiles through an iterative database search process and is capable of detecting remote homologs (Atschul et al., 1997, Nucleic Acids Res. 25: 3389-3402). Even greater sensitivity may be achieved if the family or superfamily for the peptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources, such as PSI BLAST, secondary structure prediction, structural alignment profiles, and solvation potentials, as input to a neural network that predicts the structural fold for a query sequence. Similarly, the method of Gough et al., 2000, J. Mol. Biol. 313: 903-919 may be used to align a sequence of unknown structure with the superfamily models present in the SCOP database. These alignments may be used in sequence to generate homology models for the peptides, and such models may be assessed for accuracy using a variety of tools developed for that purpose.

For proteins of known structure, several tools and resources are available for retrieving and generating structural alignments. For example, the SCOP superfamilies of proteins have been structurally aligned, and those alignments are accessible and downloadable. Two or more protein structures may be aligned using a variety of algorithms such as the distance alignment matrix (Holm and Sander, 1998, Proteins 33: 88-96) or combinatorial extension (CE) (Shindyalov and Bourne, 1998, Protein Engineering 11: 739-747), and implementation of these algorithms may be additionally utilized to query structure databases with a structure of interest in order to discover possible structural homologs (Holm and Park, 2000, Bioinformatics 16: 566-567).

The above methods are illustrative, and are not limited thereto.

Hereinafter, the specific embodiments of the present disclosure will be described in more detail.

As used herein, xylanase refers to an enzyme that catalyzes endohydrolysis of 1,4-beta-D-xylosidic bonds in xylan. For example, it may be an enzyme categorized into the glycoside hydrolase family GH11. For example, it may be endo-1,4,-beta-xylanase. For example, it may be an enzyme having an EC number of 3.2.1.8, but is not limited thereto.

In the present disclosure, xylanase activity may be measured and evaluated using methods known in the art, including the embodiments described herein.

As used herein, the term "parent xylanase" refers to xylanase to which a modification is applied in order to produce the variant or the modified polypeptide of the present disclosure. Specifically, the parent xylanase, parent enzyme, or parent sequence may be a naturally occurring polypeptide or a wild-type polypeptide, may be a mature polypeptide thereof, and may include a variant or functional fragment thereof, but is not limited thereto, as long as the polypeptide has xylanase activity and may be a parent of the variant.

The parent xylanase provided in the present disclosure may be a polypeptide of SEQ ID NO: 1, but is not limited thereto. Further, it may be a polypeptide having a sequence identity of about 60%, 70%, 75%. 80%. 85%, 90%, 95%, 96%, 97%, 98% or 99% or more with the polypeptide of SEQ ID NO: 1 as long as it has xylanase activity, and any polypeptide may be included within the scope of the parent xylanase as long as it has the same or corresponding activity to the polypeptide consisting of the amino acid sequence of SEQ ID NO: 1.

The parent xylanase of the variant provided herein may be derived from *Trichoderma* sp., *Coniochaeta* sp., *Piromyces* sp., or *Neurospora* sp. Specifically, it may be derived from *Trichoderma* sp.

Meanwhile, the above-described microorganism is an exemplary microorganism from which the parent xylanase provided herein may be derived, and includes those derived from microorganisms taxonomically homologous to the microorganism regardless of the name of the microorganisms.

The above-described microorganism may be obtained from known microorganism depositary authorities such as ATCC, DSMZ, CBS, NRRL, KCTC, and KCCM.

As used herein, a sequence "derived from" a specific microorganism is not limited to those naturally produced or producible in that microorganism, but also includes sequences encoded by genes which are produced and isolated from the microorganism containing the genes.

For example, xylanase derived from *Trichoderma* sp. may include not only enzymes having xylanase activity naturally produced by the microorganisms of *Trichoderma* sp., but also those produced in *Trichoderma* source, and those produced in other host cells through genetic modifications known in the art (e.g., transformation into a sequence encoding the enzyme).

As used herein, the "modified polypeptide having xylanase activity" may be a variant of the parent xylanase.

As used herein, the term "variant of the parent xylanase" or "xylanase variant" refers to a protein having xylanase activity with one or more amino acids different from the amino acid sequence of the parent xylanase.

The "modified polypeptide having xylanase activity", "variant of the parent xylanase" and "xylanase variant" may be used interchangeably.

The variant provided in the present disclosure may include one or more amino acid modifications in the sequences of the parent xylanase, while having xylanase activity. Further, i) the variant may be a polypeptide having a sequence identity of 70% or more and less than 100% with SEQ ID NO: 1; and/or ii) the variant may be a polypeptide encoded by a polynucleotide having a sequence identity of 70% or more and less than 100% with the sequence encoding a mature polypeptide of SEQ ID NO: 1; and/or iii) the variant may be a polypeptide encoded by (a) a mature polypeptide coding sequence of SEQ ID NO: 1, (b) a cDNA thereof, or (c) a polynucleotide that hybridizes to the full-length complement of (a) or (b) under low stringency conditions, medium stringency conditions, medium-high stringency conditions, high stringency conditions, or very high stringency conditions; and/or iv) the variant may be a functional fragment of i), ii) or iii) polypeptide having xylanase activity.

Specifically, the variant provided in the present disclosure includes modification of one or more amino acids in the parent xylanase sequence while having xylanase activity, thereby having one or more modified functions or properties, compared to the parent xylanase.

In one embodiment, the variant provided in the present disclosure includes modification of one or more amino acids in the parent xylanase sequence while having xylanase activity, thereby having one or more modified functions or properties, compared to the parent xylanase, and one or more conservative substitutions.

The variant provided in the present disclosure, which is a variant of the parent xylanase, may be a polypeptide having xylanase activity.

In one embodiment, the variant provided in the present disclosure may include modifications at one or more positions corresponding to positions 2, 3, 28, 38, 56, 62, 65, 80, 93, 114, 144, 146, 149, 151, 161, and 165 of SEQ ID NO: 1.

In the present disclosure, the position number is a position corresponding to the position of the polypeptide of SEQ ID NO: 1, and the term "corresponding" is as described above.

In any one embodiment among the above-described embodiments, the variant provided in the present disclosure may include modifications of amino acids corresponding to one or more of T2, I3, T28, N38, K56, F62, S65, S80, F93, V114, H144, S146, S149, N151, Q161, T165 of SEQ ID NO: 1.

In any one embodiment among the above-described embodiments, before the modification of the variant provided in the present disclosure, the amino acid at position 3 of SEQ ID NO: 1 may be isoleucine (I); the amino acid at position 56 may be lysine (K); the amino acid at position 62 may be phenylalanine (F); and/or the amino acid at position 146 may be serine (S).

In any one embodiment among the above-described embodiments, the amino acid corresponding to position 3 of SEQ ID NO: 1 of the variant provided in the present disclosure may be G, A, V, L, I, M, F, W, P, S, T, C, Y, N, Q, D, E, K, R, or H, specifically, G, A, V, L, I, M, F, W, P, C, Y, S, T, N, or Q, and more specifically, I, T, or V.

In any one embodiment among the above-described embodiments, the amino acid corresponding to position 56 of SEQ ID NO: 1 of the variant provided in the present disclosure may be G, A, V, L, I, M, F, W, P, S, T, C, Y, N, Q, D, E, K, R, or H, specifically, D, E, K, R, or H, and more specifically, K or R.

In any one embodiment among the above-described embodiments, the amino acid corresponding to position 62 of SEQ ID NO: 1 of the variant provided in the present disclosure may be G, A, V, L, I, M, F, W, P, S, T, C, Y, N, Q, D, E, K, R, or H, specifically, G, A, V, L, I, M, F, W, P, S, T, C, Y, N, or Q, and more specifically, F or Y.

In any one embodiment among the above-described embodiments, the amino acid corresponding to position 146 of SEQ ID NO: 1 of the variant provided in the present disclosure may be G, A, V, L, I, M, F, W, P, S, T, C, Y, N, Q, D, E, K, R, or H, specifically, G, A, V, L, I, M, F, W, P, C, Y, S, T, N, or Q, and more specifically, S or T.

In any one embodiment among the above-described embodiments, the variant provided in the present disclosure may include substitution of the amino acid corresponding to position 3 of SEQ ID NO: 1 with G, A, V, L, I, M, F, W, P, S, T, C, Y, N, Q, D, E, K, R, or H, specifically, substitution with G, V, L, I, M, F, W, P, C, Y, S, T, N, or Q, and more specifically, substitution with T or V.

In any one embodiment among the above-described embodiments, the variant provided in the present disclosure may include substitution of the amino acid corresponding to position 56 of SEQ ID NO: 1 with G, A, V, L, I, M, F, W, P, S, T, C, Y, N, Q, D, E, R, or H, specifically, substitution with R or H, and more specifically, substitution with R.

In any one embodiment among the above-described embodiments, the variant provided in the present disclosure may include substitution of the amino acid corresponding to position 62 of SEQ ID NO: 1 with G, A, V, L, I, M, W, P, S, T, C, Y, N, Q, D, E, K, R, or H, specifically, substitution with W or Y, and more specifically, substitution with Y.

In any one embodiment among the above-described embodiments, the variant provided in the present disclosure may include substitution of the amino acid corresponding to position 146 of SEQ ID NO: 1 with G, A, V, L, I, M, F, W, P, T, C, Y, N, Q, D, E, K, R, or H, specifically, substitution with C, Y, T, N, or Q, and more specifically, substitution with T.

In any one embodiment among the above-described embodiments, the variant provided in the present disclosure may include substitution of the amino acid corresponding to position 3 of SEQ ID NO: 1 with a nonpolar or polar amino acid.

In any one embodiment among the above-described embodiments, the variant provided in the present disclosure may include substitution of the amino acid corresponding to position 56 of SEQ ID NO: 1 with a basic amino acid.

In any one embodiment among the above-described embodiments, the variant provided in the present disclosure may include substitution of the amino acid corresponding to position 62 of SEQ ID NO: 1 with an aromatic amino acid.

In any one embodiment among the above-described embodiments, the variant provided in the present disclosure may include substitution of the amino acid corresponding to position 146 of SEQ ID NO: 1 with a polar amino acid.

In any one embodiment among the above-described embodiments, the variant provided in the present disclosure may include substitution of one or more of I3T/V, K56R, F62Y and S146T of SEQ ID NO: 1.

In any one embodiment among the above-described embodiments, the variant provided in the present disclosure may include substitution of one or more positions, for example, 2, 3, 4, 5, 6, 7, 8, 9, 10, 11, 12, 13, 14, or 15 positions selected from positions 2, 3, 28, 38, 56, 62, 65, 80, 93, 114, 144, 146, 149, 151, 161, and 165. For example, the variant may be a variant represented by SEQ ID NO: 5, SEQ ID NO: 17, SEQ ID NO: 19, SEQ ID NO: 21, SEQ ID NO: 23 or SEQ ID NO: 25.

Specifically, the variant including I3T + K56R + F62Y + S146T + T2C + T28C + N38D + S65T + S80T + F93Y + V114T + H144K + S149T + N151T + Q161R + T165V substitutions of SEQ ID NO: 1 may be represented by SEQ ID NO: 5, the variant including T3I substitution of SEQ ID NO: 5 may be represented by SEQ ID NO: 17, the variant including T3V substitution of SEQ ID NO: 5 may be represented by SEQ ID NO: 19, the variant including R56K substitution of SEQ ID NO: 5 may be represented by SEQ ID NO: 21, the variant including Y62F substitution of SEQ ID NO: 5 may be represented by SEQ ID NO: 23, and the variant including T146S substitution of SEQ ID NO: 5 may be represented by SEQ ID NO: 25.

In one embodiment, the variant provided in the present disclosure includes all possible combinations of the modifications described above.

For example, the variant may include modifications of amino acids at positions selected below, by combinations of the modifications described above:
position 3;
position 56;
position 62;
position 146;
positions 3 + 56;
positions 3 + 62;
positions 3 + 146;
positions 56 + 62;
positions 56 + 146;
positions 62 + 146;
positions 3 + 56 + 62;
positions 3 + 56 + 146;
positions 3 + 62 + 146;
positions 56 + 62 + 146; and
positions 3 + 56 + 62 + 146.

In another example, the variant may include any one or more substitutions of the following i) to iv), but is not limited thereto: i) substitution of the amino acid at position 3 with threonine; ii) substitution of the amino acid at position 56 with arginine; iii) substitution of the amino acid at position 62 with tyrosine; and iv) substitution of the amino acid at position 146 with threonine.

In still another example, the variant may include substitutions of 15 or more positions of positions 2, 3, 28, 38, 56, 62, 65, 80, 93, 114, 144, 146, 149, 151, 161, and 165, but is not limited thereto.

In still another example, the variant may include any one or more selected from the following modifications, but is not limited thereto:
I3T/V ;
K56R ;
F62Y ;
S146T ;
I3T/V + K56R ;
I3T/V + F62Y ;
I3T/V + S146T ;
K56R + F62Y ;
K56R + S146T;
F62Y + S146T;
I3T/V + K56R + F62Y ;
I3T/V + K56R + S146T ;
I3T/V + F62Y + S146T ;
K56R + F62Y + S146T ;
I3T/V + K56R + F62Y + S146T ;
I3T/V + T2C + T28C + N38D + S65T + S80T + F93Y + V114T + H144K + S149T + N151T + Q161R + T165V;
K56R + T2C + T28C + N38D + S65T + S80T + F93Y + V114T + H144K + S149T + N151T + Q161R + T165V;
F62Y + T2C + T28C + N38D + S65T + S80T + F93Y + V114T + H144K + S149T + N151T + Q161R + T165V;
S146T + T2C + T28C + N38D + S65T + S80T + F93Y + V114T + H144K + S149T + N151T + Q161R + T165V;
I3T/V + K56R + T2C + T28C + N38D + S65T + S80T + F93Y + V114T + H144K + S149T + N151T + Q161R + T165V;
I3T/V + F62Y + T2C + T28C + N38D + S65T + S80T + F93Y + V114T + H144K + S149T + N151T + Q161R + T165V;
I3T/V + S146T + T2C + T28C + N38D + S65T + S80T + F93Y + V114T + H144K + S149T + N151T + Q161R + T165V;
K56R + F62Y + T2C + T28C + N38D + S65T + S80T + F93Y + V114T + H144K + S149T + N151T + Q161R + T165V;
K56R + S146T + T2C + T28C + N38D + S65T + S80T + F93Y + V114T + H144K + S149T + N151T + Q161R + T165V;
F62Y + S146T + T2C + T28C + N38D + S65T + S80T + F93Y + V114T + H144K + S149T + N151T + Q161R + T165V;
I3T/V + K56R + F62Y + T2C + T28C + N38D + S65T + S80T + F93Y + V114T + H144K + S149T + N151T + Q161R + T165V;
I3T/V + K56R + S146T + T2C + T28C + N38D + S65T + S80T + F93Y + V114T + H144K + S149T + N151T + Q161R + T165V;
I3T/V + F62Y + S146T + T2C + T28C + N38D + S65T + S80T + F93Y + V114T + H144K + S149T + N151T + Q161R + T165V;
K56R + F62Y + S146T + T2C + T28C + N38D + S65T + S80T + F93Y + V114T + H144K + S149T + N151T + Q161R + T165V; and
I3T/V + K56R + F62Y + S146T + T2C + T28C + N38D + S65T + S80T + F93Y + V114T + H144K + S149T + N151T + Q161R + T165V.

In one embodiment, the variant provided in the present disclosure may have a sequence identity of about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% with the parent xylanase; a mature polypeptide thereof, or a functional fragment thereof.

In one embodiment, the variant provided in the present disclosure may have a sequence identity of about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% with SEQ ID NO: 1.

In one embodiment, the variant provided in the present disclosure may be a polypeptide encoded by a polynucleotide having a sequence identity of about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% with a sequence encoding the mature polypeptide of SEQ ID NO: 1.

In one embodiment, the variant provided in the present disclosure may have a sequence identity of about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% with a functional fragment of SEQ ID NO: 1.

In the variant provided in the present disclosure, one or more of any selectable or detectable properties or attributes of a polypeptide may be altered as compared to other xylanases, e.g., a wild-type xylanase, parent xylanase, other xylanase variants, etc.

The properties or attributes may include, but are not limited to, oxidative stability, substrate specificity, catalytic activity, thermal stability, alkaline stability, pH activity profile, resistance to proteolytic degradation, Km, k_{cat}, k_{cat}/Km ratio, protein folding, inducing an immune response, ability to bind to a ligand, ability to bind to a receptor, ability to be secreted, ability to be displayed on the cell surface, ability to oligomerize, ability to signal, ability to stimulate cell proliferation, ability to inhibit cell proliferation, ability to induce apoptosis, ability to be modified by phosphorylation or glycosylation, and/or ability to treat diseases, etc.

Specifically, the variant provided in the present disclosure may have increased thermal tolerance and/or thermal stability compared to the parent sequence.

As used herein, the "enzymatic activity" exhibits at least one catalytic activity. Specifically, it may be the conversion efficiency of an enzyme, which is mainly expressed as k_{cat}/Km, but is not limited thereto.

When the enzyme is completely saturated with substrates, k_{cat} means the rate constant (catalytic constant) that converts substrates into products in unit time by one enzyme, and is also referred to as a turnover number. Km is the substrate concentration when the reaction rate is at half the maximum value (Vmax).

Examples of the ways to express enzymatic activity include specific activity (umol of converted substrate x mg ⁻¹ x min ⁻¹) or volumetric activity (umol of converted substrate x mL ⁻¹ x min ⁻¹), etc.

However, defining the enzymatic activity is not limited to the description described above, and the enzymatic activity may be defined and evaluated, based on the information disclosed in Irwin H. Segel, Enzyme kinetics, John Wiley & Sons, 1979; A. G. Marangoni, Enzyme kinetics, Wiley-Interscience, 2003; A. Fersht, Enzyme structure and mechanisms, John Wiley & Sons, 1981; Structure and Mechanism in Protein Science: A guide to enzyme catalysis and protein folding, Alan Fersht, W.H. Freeman, 1999; Fundamentals of Enzyme Kinetics, Athel Cornish-Bowden, Wiley-Blackwell 2012 and Voet ef al., "Biochemie" [Biochemistry], 1992, VCH-Verlag, Chapter 13, pages 331-332 with respect to enzymatic activity, etc.

In one embodiment, the variant provided in the present disclosure may have an increased enzymatic activity by about 100%, about 110%, about 120%, about 130%, about 140%, about 150%, about 160%, about 170%, about 180%, about 190%, about 200%, about 300%, about 400%, about 500%, about 530%, about 550%, about 600%, about 620%, about 650%, about 700%, about 720%, about 770%, about 800%, about 820%, about 830% or more, as compared to the parent enzyme. In any one embodiment among the above-described embodiments, the parent enzyme may be the polypeptide represented by SEQ ID NO: 1.

As used herein, the term "specific activity" is the activity of an enzyme per unit weight of proteins, and may be expressed as unit/mg. The quantification of proteins may be performed using, for example, SDS-PAGE or Bradford assay.

Enzyme stability means that enzyme activity is maintained during storage or reaction time. In order to measure such changes in stability, the initial enzyme activity may be measured and compared under defined conditions at time zero (100%) and after a predetermined period of time (x%), and thus, the level at which the enzyme activity is lost or the enzyme stability may be expressed.

Factors that affect enzyme activity include, for example, pH, heat, the presence of other substances (e.g., oxidizing agents, chelating agents), etc.

As used herein, the term "pH stability" means the ability of a protein to function in a specific pH range. In one embodiment, the variant provided in the present disclosure may have activity at about pH 3.0 to about pH 8.0, but is not limited thereto.

When a protein maintains its function in a specific pH range, it may be defined as having "pH stability", and may also be defined as having "acid resistance", "alkali resistance", etc., according to the pH range.

As used herein, the term "thermal stability" means the ability of a protein to function in a specific temperature range. In one embodiment, the variant provided in the present disclosure may have activity in the range of about 25°C to about 100°C, but is not limited thereto.

As used herein, the term "thermal tolerance" means the ability of a protein to function after exposure to a specific temperature, e.g., high heat or cryogenic temperature. For example, proteins having thermal tolerance may not function at a temperature they are exposed to, but may become functional when returned to an optimal temperature environment.

An increase in stability may include maintaining high enzymatic activity; increasing the range of pH, temperature and/or time, etc., at which a protein maintains its function, by comparing to other enzymes, e.g., a wild-type enzyme, a parent enzyme, and/or other variants.

A decrease in stability may include maintaining low enzymatic activity; decreasing the range of pH, temperature and/or time, etc., at which a protein maintains its function, by comparing to other enzymes, e.g., a wild-type enzyme, a parent enzyme, and/or other variants.

As used herein, the term "substrate specificity" means the ability of an enzyme to identify a substrate and molecules that compete with the substrate. Substrate specificity may be determined by measuring the activity of an enzyme for different substrates. In one embodiment, the change in substrate specificity may be a change in the direction of increasing specificity for a substrate capable of producing a desired product. In another embodiment, the change in substrate specificity may be a change in a direction of decreasing specificity for a substrate capable of producing a desired product.

The altered properties of the variant provided in the present disclosure may be suitable or improved activity for application in various industrial fields, including feeds, baking, pulp bleaching, etc.

The polynucleotide encoding the variant of the present disclosure may include the coding sequence of the above-described variant. The polynucleotide may undergo various modifications in the coding region within the scope that does not change the amino acid sequence of the polypeptide, due to codon degeneracy or in consideration of the codons preferred in an organism in which the polypeptide is to be expressed.

Further, the polynucleotide of the present disclosure may include a probe that may be prepared from a known gene sequence, for example, any sequence encoding the variant of the present disclosure by hybridizing with a sequence complementary to all or part of the nucleotide sequence described above under stringent conditions without limitation.

The "stringent conditions" refers to conditions under which specific hybridization between polynucleotides is allowed. Such conditions are specifically described in the literature (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

For example, the stringent conditions may include conditions under which polynucleotides having a high homology or identity of 40% or more, specifically 90% or more, more specifically 95% or more, 96% or more, 97% or more, 98% or more, and even more specifically 99% or more are hybridized with each other and polynucleotides having a homology or identity lower than the above homologies or identities are not hybridized with each other, or washing conditions of the common Southern hybridization, that is, washing once, specifically, twice or three times at a salt concentration and a temperature corresponding to 60°C, 1XSSC, 0.1% SDS, specifically, 60°C, 0.1XSSC, 0.1% SDS, and more specifically 68°C, 0.1 × SSC, 0.1% SDS.

Hybridization requires that two nucleic acids contain complementary sequences, although mismatches between bases are possible depending on the stringency of the hybridization. The term "complementary" is used to describe the relationship between nucleotide bases that may hybridize with each other. For example, with respect to DNA, adenine is complementary to thymine, and cytosine is complementary to guanine. Therefore, the polynucleotide of the present disclosure may include isolated nucleotide fragments complementary to the entire sequence as well as nucleic acid sequences substantially similar thereto.

Specifically, polynucleotides having a homology or identity may be detected using the hybridization conditions including a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (see, Sambrook et al., supra, 9.50-9.51, 11.7-11.8).

For example, the "high stringency" may occur at about 5°C to 10°C below the Tm of the probe; the "medium stringency" may occur at about 10°C to 20°C below the Tm of the probe; and the "low stringency" may occur at about 20°C to 25°C below the Tm, but the stringency is not limited thereto.

In one example, the "low stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 25% formamide, according to the standard Southern blotting procedures for 12 hours to 24 hours. The carrier material may be finally washed twice or three times each for 15 minutes in 2×SSC, 0.1% SDS to 0.2% SDS at 50°C.

In one example, the "medium stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 hours to 24 hours. The carrier material may be finally washed twice or three times each for 15 minutes in 2×SSC, 0.1 % SDS to 0.2% SDS at 55°C. In one example, the "medium-high stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 hours to 24 hours. The carrier material may be finally washed twice or three times each for 15 minutes in 1 × SSC to 2 × SSC, 0.1 % SDS to 0.2% SDS at 60°C.

In one example, the "high stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 hours to 24 hours. The carrier material may be finally washed twice or three times each for 15 minutes in 2×SSC, 0.1% SDS to 0.2% SDS at 65°C.

The nucleic acid construct provided in the present disclosure may include a polynucleotide encoding the variant provided herein, operably linked to one or more regulatory sequences that direct the expression of the coding sequence in an appropriate host cell under conditions suitable for the regulatory sequences.

The polynucleotides may be engineered in various ways to allow the expression of variants. Depending on the expression vector, it may be desirable or necessary to manipulate the polynucleotides prior to insertion into the vector. Such manipulations may be performed using methods known in the art.

The "vector" provided in the present disclosure refers to a DNA construct containing the nucleotide sequence of the polynucleotide encoding the variant operably linked to a suitable expression regulatory region (expression regulatory sequence) so as to be able to express the variant of the present disclosure in a suitable host cell. The expression regulatory region may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into genome thereof.

The vector that may be used in the present disclosure is not particularly limited, and any vector known in the art may be used. Examples of the vectors commonly used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, etc. may be used; and as a plasmid vector, those based on pBR, pUC, pBluescriptII, pGEM, pTZ, pCL and pET, etc. may be used. Specifically, pDZ, pACYC177, pACYC184, pCL, pECCG117, pUC19, pBR322, pMW118, pCC1BAC vector, etc. may be used.

For example, the polynucleotide encoding the variant provided in the present disclosure may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or show different phenotypes under the environment treated with the selective agent, and thus the transformed cells may be selected.

Any host cell may be included in the host cell of the present disclosure as long as it may express the variant of the present disclosure without limitation.

The host cell of the present disclosure may include the above-described variant, a polynucleotide encoding the variant, a nucleic acid construct and/or vector including the same.

The nucleic acid construct or vector may be integrated into the chromosome as described earlier, or may remain as a self-replicating extrachromosomal vector.

The host cell of the present disclosure encompasses any progeny of a parent cell that is not identical to the parent cell due to mutations that occur during replication.

The host cell may be any cell useful in the recombinant production of the variant, e.g., a prokaryotic or eukaryotic cell.

The prokaryotic host cell may be any Gram-positive or Gram-negative bacterium.

The Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.*

The Gram-negative bacteria include, but are not limited to, *Campylobacter, Escherichia coli, Flavobacterium, Fusobacterium, Helicobacter, Ilyobacter*, *Neisseria, Pseudomonas, Salmonella, Vibrio* (e.g., Vibrio natriegens), and *Ureaplasma.*

In one embodiment, the bacterial host cell may be a host cell belonging to the genus *Bacillus,* specifically including *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells, but is not limited thereto.

In one embodiment, the bacterial host cell may be a host cell belonging to the genus *Streptococcus,* specifically including *Streptococcus equisimilis, Streptococcus pyogenes, Streptococcus uberis,* and *Streptococcus equi subsp. Zooepidemicus,* but is not limited thereto.

In one embodiment, the bacterial host cell may be a host cell belonging to the genus *Streptomyces,* specifically including *Streptomyces achromogenes, Streptomyces avermitilis, Streptomyces coelicolor, Streptomyces griseus,* and *Streptomyces lividans*, but is not limited thereto.

In one embodiment, the bacterial host cell may be a host cell belonging to the genus *Corynebacterium,* and may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* but is not limited thereto.

The host cell may be a eukaryote, such as a mammalian, insect, plant, or fungal cell.

The host cell may be a fungal cell. As used herein, the "fungi" include Ascomycota, Basidiomycota, Chytridiomycota, and Zygomycota as well as the Oomycota and all mitosporic fungi.

The fungal host cell may be a yeast cell. As used herein, the "yeast" includes ascosporogenous yeast (*Endomycetales*), basidiosporogenous yeast, and yeast belonging to the Fungi imperfecti (*Blastomycetes*). However, the classification of yeasts may change, and may be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Komagataella, Saccharomyces, Schizosaccharomyces,* or *Yarrowia* cell, such as *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, Komagataella phaffii,* or *Yarrowia lipolytica.*

The fungal host cell may be a filamentous fungal cell. The "filamentous fungi" include all filamentous forms of the subdivision *Eumycota* and *Oomycota* (as defined in the literature (Hawksworth et al., 1995)). The filamentous fungi are generally characterized by a mycelial wall composed of chitin, cellulose, glucan, chitosan, mannan, and other complex polysaccharides. Vegetative growth is by hyphal elongation, and carbon catabolism is obligately aerobic. In contrast, vegetative growth by yeasts such as *Saccharomyces cerevisiae* is by budding of a unicellular thallus, and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix*, *Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides*, *Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides*, *Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phiebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride,* but is not limited thereto.

The method of preparing the variant of the present disclosure may include the step of culturing the host cell.

As used herein, the term "culturing" means that the host cell is grown under appropriately controlled environmental conditions. The culturing process of the present disclosure may be performed in a suitable medium and culture conditions known in the art. Such a culturing process may be easily adjusted for use by those skilled in the art according to the strain to be selected. Specifically, the culturing may be a batch culture, a continuous culture, and a fed-batch culture, but is not limited thereto.

As used herein, the term "medium" refers to a mixture of materials which contains nutrient materials required for the culturing of the host cell as a main ingredient, and it supplies nutrient materials and growth factors, along with water that is essential for survival and growth. Specifically, the medium and other culture conditions used for culturing the host cell of the present disclosure may be any medium used for common culturing cultivation of host cells without any particular limitation. However, the host cell of the present disclosure may be cultured under aerobic conditions in a common medium containing an appropriate carbon source, nitrogen source, phosphorus source, inorganic compound, amino acid, and/or vitamin, while adjusting temperature, pH, etc.

In the present disclosure, the carbon source may include carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; amino acids, such as glutamic acid, methionine, lysine, etc. Further, natural organic nutrients such as starch hydrolysate, molasses, blackstrap molasses, rice bran, cassava, sugar cane molasses, and corn steep liquor, etc. may be used. Specifically, carbohydrates such as glucose and sterilized pretreated molasses (i.e., molasses converted to reducing sugar) may be used, and in addition, various other carbon sources may be used in an appropriate amount without limitation. These carbon sources may be used alone or in a combination of two or more thereof, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extract, yeast extract, malt extract, corn steep liquor, casein hydrolysate, fish or decomposition product thereof, defatted soybean cake or decomposition product thereof, etc. These nitrogen sources may be used alone or in a combination of two or more thereof, but are not limited thereto.

The phosphorus source may include monopotassium phosphate, dipotassium phosphate, or corresponding sodium-containing salts, etc. Examples of the inorganic compounds may include sodium chloride, calcium chloride, iron chloride, magnesium sulfate, iron sulfate, manganese sulfate, calcium carbonate, etc. In addition, amino acids, vitamins, and/or appropriate precursors may be included. These components or precursors may be added to a medium in a batch or continuous manner, but are not limited thereto.

Further, pH of the medium may be adjusted by adding a compound such as ammonium hydroxide, potassium hydroxide, ammonia, phosphoric acid, sulfuric acid, etc. during the culturing of the host cell in an appropriate manner. In addition, bubble formation may be prevented during the culturing using an antifoaming agent such as fatty acid polyglycol ester. Further, oxygen gas or an oxygen-containing gas may be injected to the medium in order to maintain aerobic conditions of the medium; or no gas may be injected, or nitrogen, hydrogen, or carbon dioxide gas may be injected in order to maintain anaerobic or microaerobic conditions, but the gas is not limited thereto.

The temperature of the medium may be 20°C to 55°C, specifically, 25°C to 40°C, but is not limited thereto. The culturing may be continued until the desired amount of a useful material is obtained, and may be specifically carried out for 24 hours to 196 hours, but is not limited thereto.

In one embodiment, the method of preparing the modified polypeptide having xylanase activity of the present disclosure may further include the step of recovering the modified polypeptide having xylanase activity of the present disclosure which is expressed in the culturing step.

In another embodiment, the variant expressed in the culturing step may be recovered using methods known in the art to which the present disclosure pertains. For example, the variant may be recovered from a nutrient medium by common procedures including, but not limited to, collection, centrifugation, filtration, extraction, spray-drying, evaporation, or precipitation.

The recovering method may be collecting the variant using the method of culturing the host cell of the present disclosure, for example, using a suitable method known in the art according to a batch culture, continuous culture, or fed-batch culture method. For example, methods such as centrifugation, filtration, treatment with a protein crystallizing precipitant (salting-out method), extraction, sonication, ultrafiltration, dialysis, various kinds of chromatographies such as molecular sieve chromatography (gel filtration), adsorption chromatography, ion exchange chromatography, affinity chromatography, etc., HPLC and a combination thereof may be used, and the variant may be recovered from the medium or the host cell using suitable methods known in the art.

In another embodiment, the variant expressed by the host cell in the culturing step may not be recovered. In the embodiment, the host cell expressing the variant may be used as a source of the variant.

The composition of the present disclosure may be used to degrade xylan-containing materials.

The composition of the present disclosure may be used to convert xylan-containing materials into xylose and/or xylo-oligosaccharides.

The composition of the present disclosure may further include other components in addition to the variant provided in the present disclosure. The components added to the composition of the present disclosure may be appropriately selected by those skilled in the art.

In one embodiment, the composition of the present disclosure may further include any components suitable for conversion of xylan-containing materials to xylose and/or xylo-oligosaccharides.

In one embodiment, the composition of the present disclosure may further include any components suitable for application in various industrial fields such as animal feeds, baking, biomass saccharification, pulp bleaching, etc.

Examples of materials that may be added include stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, etc., but are not limited thereto.

In one embodiment, the composition provided in the present disclosure may further include a naturally occurring material or a non-naturally occurring material, in addition to the variant provided in the present disclosure.

In one embodiment, the composition provided in the present disclosure may further include additional enzymes commonly used in various industrial fields, including animal feeds, baking, biomass saccharification, pulp bleaching, etc., in addition to the variant provided in the present disclosure.

For example, the additional enzymes may further include any one or more enzymes selected from the group consisting of beta-amylase, cellulase (beta-glucosidase, cellobiohydrolase and endoglucanase), glucoamylase, hemicellulase (e.g., endo-xylanase, β-xylosidase, α-L-arabinofuranosidase, α-D-glucuronidase, feruloyl esterase, coumaroyl esterase, α-galactosidase, β-galactosidase, β-mannanase or β-mannosidase), isoamylase, isomerase, lipase, phytase, protease, pullulanase, and/or other enzymes useful in a commercial process in conjunction with alpha-amylase.

The xylanase variant of the present disclosure or the composition including the xylanase variant of the present disclosure may be used to degrade any xylan-containing materials.

As used herein, the xylan-containing material is any material that may be degraded by xylanase. In one example, the xylan-containing material may be hemicellulose. Specifically, the xylan-containing material may be a material selected from xylan, glucuronoxylan, arabinoxylan, glucomannan, and xyloglucan. In one example, the xylan-containing material may be xylan, but is not limited thereto.

In one embodiment, the present disclosure provides a method of degrading (or break down) xylan-containing materials. This may also be referred to as solubilization of xylan and/or solubilization of pentosans.

In further embodiment of the present disclosure, the method relates to the degradation of polymers (e.g., break down) derived from the degradation of xylan.

The breakdown products (e.g., glucose) may be used as a feedstock for any fermentation process, such as in biofuel (e.g., bioethanol) production or in the production of other products such as biochemicals (e.g., bio-based isoprene).

Xylan may be degraded using the variant of the present disclosure, the host cell expressing the same, and/or the composition including the variant and/or the host cell. In addition to the variant of the present disclosure, cofactors, coenzymes, etc. may be added together in the hydrolysis step of xylan. The hydrolysis step of the substrate may be performed under optimal pH, temperature conditions, etc., and appropriate conditions may be selected by those skilled in the art.

The xylanase variant of the present disclosure may be used in any one of the following applications:
a) an additive in animal feedstuffs; and/or
b) a feed supplement for animals; and/or
c) breakdown of grain-based materials (e.g., this may be whole grain or part of grain).

In one embodiment, the xylanase variant of the present disclosure may be used in feedstuffs.

In one embodiment, the xylan-containing material may be a feedstuff or a feed component.

The feed composition of the present disclosure may refer to any natural or artificial diet, a single meal, etc., or a component of the single meal, which an animal eats, ingests and digests, or which is suitable for eating, ingestion and digestion, and the feed composition may be prepared in various forms known in the art.

In one embodiment, the xylanase variant of the present disclosure may be used for a food composition or the preparation thereof.

In one embodiment, the xylan-containing material may be a grain-based material (including whole or part of grains, or malted grains, e.g., malted barley).

In one embodiment, the xylan-containing material may be a cereal flour (e.g., wheat, oat, rye or barley flour).

In one embodiment the xylan-containing material may be a barley malt or mash, or malted barley or combinations thereof.

In one example, the food composition may be a fermented beverage including beer and wine. In another example, the food composition may be bakery products, including loaves, rolls, buns, pizza, pretzels, tortillas, cakes, cookies, biscuits, and crackers, but is not limited thereto.

The xylanase variant of the present disclosure may be used for wheat gluten-starch separation.

For example, after initial separation of the wheat bran and germ from the endosperm, fractionation of wheat endosperm flour into starch and gluten fractions may be employed to obtain high-quality α-starch, β-starch byproducts, and vital gluten.

In the method of separating a cereal flour (e.g., wheat flour) into starch and gluten fractions, the method includes the step of mixing the cereal flour (e.g., wheat flour), water and the xylanase variant. The cereal flour, water and xylanase variant may be mixed simultaneously or sequentially. In some embodiments, the cereal flour (e.g., wheat flour) and water may be mixed before mixing with the xylanase variant.

When the xylanase variant of the present disclosure is applied for wheat gluten-starch separation, it may produce higher α-starch yields and/or better-quality gluten (e.g., better-quality vital gluten).

In another embodiment, the xylanase variant of the present disclosure may be used for the degradation of grain-based materials and may be used as part of a biofuel (e.g., bioethanol) production process.

In one example, the xylanase variant of the present disclosure may improve the production of biofuels (e.g., bioethanol) and the use of grain-based materials in the biofuel industry.

In one example, the method may include the step of mixing a biofuel with the xylanase variant prior to or during liquefaction, saccharification, fermentation, simultaneous saccharification and fermentation, post fermentation, or a combination thereof.

When the xylanase variant of the present disclosure is applied to the biofuel production process, the following advantages may be obtained: higher content of dry substance mash may be used in the process; higher solids content of final syrup may be obtained; higher heat transfer; lower energy requirement; reduced evaporator fouling; reduced cleaning costs; increased final fuel yields; improved quality of by-product; better separation between the solid and liquid part of stillage after distillation; or a combination thereof.

The xylanase variant of the present disclosure may be used for pulp bleaching.

For example, when the xylanase variant is treated while linking the pigmented lignin in the pulp to crystalline cellulose via xylan, xylan may be degraded and the pigmented lignin may be released, thereby promoting pulp bleaching.

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1: Preparation of Xylanase Single Variants

### 1-1. Preparation of Template

A gene (SEQ ID NO: 2) encoding xylanase (hereinafter referred to as Tr Xyn, SEQ ID NO: 1) of *Trichoderma ressei* strain was amplified and cloned into a pHCE vector (Takara), which was then used as a template.

### 1-2. Preparation of Xylanase Single Variants

The position 3 of Tr Xyn was selected as a site for improvement, and primers (Table 1) for mutating the corresponding amino acid sequences were designed using the Tr Xyn sequence inserted into the pHCE vector as a template to prepare saturated mutagenesis.

In detail, the xylanase single variants were prepared by PCR using the template, primers, and a PCR premix (iNtRON, cat no. 25185). PCR was performed using Eppendorf Mastercycler Nexus GX2, and the reaction conditions are as follows:
Initial denaturation: 94°C, 2 min
Denaturation: 94°C, 20 sec
Annealing: 55°C, 10 sec
Extension: 72°C, 10 min (30 cycles from denaturation to extension)
Final Extension: 72 °C, 5 min

The prepared 19 kinds of single variants were ligated using an In-Fusion HD cloning kit (Takara, Cat. No. 639650), and then transformed into an *E*. *coli* DH5α strain, followed by sequencing to confirm sequence mutation.

**[Table 1]**

| Name of Variant | Mutation Description | | SEQ ID NO: | Primer (5' → 3') |
|---|---|---|---|---|
| Tr Xyn single variant | Saturated mutagenesis at position 3 | 3I_SSM_Forward | 3 | CAACCAGGCACAGGATACAACAATGGCTA CTT |
| | | 3I_SSM_Reverse | 4 | TGTGCCTGGTTGMNNAGTCTGCATATGGAT ATCTCCTTTTTCCAG |

### 1-3. Selection of Single Variant to Improve Thermal Tolerance of Xylanase

The *E.coli* BL21(DE3) strains each transformed with the single variant prepared in Example 1-2 and Tr Xyn gene were inoculated into a sterile LB kanamycin medium (BD Difco) in a 96-deep well plate (Bioneer, 90060), and then cultured at 37°C and 800 rpm 24 hours, and then inoculated into a main medium in a 96-deep well plate. After the cells were recovered through centrifugation, the recovered cells were treated with BugBuster (Merck, 70584) and disrupted. The disrupted enzyme solution was centrifuged, and only the supernatant was recovered, and left at each temperature to evaluate thermal tolerance.

As a method of measuring thermal tolerance of enzymes, 90 µl of 0.5% xylan from beachwood (Megazyme, P-XYLNBE-10G) dissolved in a citrate buffer at pH 5.5 was mixed with 20 µl of the heat-treated enzyme solution and then allowed to react at 37°C. Then, the reaction solution was mixed with 150 µl of a DNS solution to stop the reaction, and the color was developed in a dry oven at 80°C. The absorbance was measured at 540 nm, and the activity was measured using a standard curve obtained with xylose (Sigma-Aldrich, X1500).

A method of preparing the DNS solution is as follows: 15.75 g of 3,5-dinitrosalicylic acid (samchun, D1267) was added to a beaker containing 2500 ml of distilled water, the temperature was adjusted to 45°C, and then 500 ml of 20% sodium hydroxide solution (Daejung, 7571-4400) was added. 455 g of potassium sodium tartrate tetrahydrate (Daejung, 6618-4400) was added, and then 12.5 g of phenol (Sigma-Aldrich, P1037) and 12.5 g of sodium sulfite anhydrous (Daejung, 7634-4405) were added and the temperature was adjusted to 45°C, followed by stirring. Thereafter, 1500 ml of distilled water was added, stirring was performed until dissolved, followed by cooling. Distilled water was added thereto up to 5000 ml, filtered, and stored in a brown bottle for 7 days or more before use.

Residual activity was evaluated to confirm thermal stability of variants, in which the amino acid at position 3 was modified with various amino acids. As a result, it was confirmed that when the amino acid at position 3 was substituted with valine (Val), an increase in thermal stability was about 250%, compared to the wild strain (Table 2).

**[Table 2]**

| Substituted amino acid | Thermal stability relative to WT (%) |
|---|---|
| I(WT) | 100.0 |
| V | 259.0 |
| Y | 0.8 |
| Q | 47.7 |
| G | 0.9 |
| R | 0.7 |
| S | 6.7 |
| H | 1.8 |
| T | 58.0 |
| L | 83.9 |
| N | 0.0 |
| M | 67.0 |
| A | 3.8 |
| P | 0.2 |
| D | 2.6 |
| F | 0.0 |
| C | 77.8 |
| W | 3.1 |
| K | 0.0 |
| E | 88.8 |

### Example 2: Preparation of Xylanase Combination Variants

### 2-1. Preparation of Xylanase Combination Variants

As a result of identifying mutations in the Tr Xyn multiple variant with an amino acid sequence of SEQ ID NO: 5, which was confirmed to have excellent thermal tolerance, the amino acid at position 3 was substituted with T, and positions 56, 62, and 146 were newly identified. Accordingly, to evaluate the effect on thermal tolerance through a restoration experiment, a variant was prepared. In order to also confirm the 13V effect which was confirmed in Example 1-3, variants were prepared, each in which the amino acid at position 3 was restored or substituted with V (Table 3).

**[Table 3]**

| Name of variant | Mutation description | | SEQ ID NO. | Primer (5' → 3') |
|---|---|---|---|---|
| Multiple variant_T3I | T3I | Forward | 7 | CAACCTGGTACAGGGTACAATAATGGTT AC |
| | | Reverse | 8 | TGTACCAGGTTGAATACACTGCATATGTA TATCTCCTTCTTAAAG |
| Multiple variant_T3V | T3V | Forward | 9 | CAACCTGGTACAGGGTACAATAATGGTT AC |
| | | Reverse | 10 | TGTACCAGGTTGCACACACTGCATATGT ATATCTCCTTCTTAAAG |
| Multiple variant_R56K | R56K | Forward | 11 | AATAAGGTGATTAACTACAGTGGGACTTA CA |
| | | Reverse | 12 | AATCACCTTATTTTTAGTTCCGGGCTGCC ACC |
| Multiple variant_Y62F | Y62F | Forward | 13 | AGTGGGACTTACAACCCAAATGGC |
| | | Reverse | 14 | GTAAGTCCCACTAAAGTTAATCACCTTAT TGCGAG |
| Multiple variant_T146S | T146S | Forward | 15 | TCTGGTACTGTAACAACTGCGAACCA |
| | | Reverse | 16 | TACAGTACCAGAAGAGCGCTTGTTACGA CGTA |

In detail, the xylanase variants were prepared by PCR using the template, primers, and a PCR premix (iNtRON, cat no. 25185), or a QuikChange Site-Directed Mutagenesis Kit (Agilent technologies, cat no. 200518). PCR was performed using Eppendorf Mastercycler Nexus GX2, and the reaction conditions are as follows:
Initial denaturation: 94°C, 2 min
Denaturation: 94°C, 20 sec
Annealing: 55°C, 10 sec
Extension: 72°C, 10 min (30 cycles from denaturation to extension)
Final Extension: 72 °C, 5 min

The prepared 5 kinds of variants were ligated using an In-Fusion HD cloning kit (Takara, Cat. No. 639650), and then transformed into an E. *coli* DH5α strain, followed by sequencing to confirm sequence mutation.

### 2-2. Selection of Combination Variants with Improved Xylanase Thermal Tolerance

The *E.coli* BL21(DE3) strains, each transformed with the combination variants prepared in Example 2-1 and the Tr Xyn gene, were each inoculated into a sterile LB medium (BD Difco), and cultured for 24 hours at 37°C and 180 rpm, and then the cells were recovered by centrifugation. 20 ml of a lysis buffer (50 mM Tris-HCl pH 8.0, 100 mM NaCl, 10 mM imidazole) was added to the recovered cells and resuspended, and then a crude enzyme solution was obtained through sonication and centrifugation. The crude enzyme solution was applied to a Ni-NTA resin (Qiagen, Cat no. 30230) to be adsorbed, followed by sequentially applying a washing buffer (only 20 mM, 50 mM, 110 mM of imidazole in the lysis buffer composition) and an elution buffer (only 250 mM of imidazole in the lysis buffer composition) to purify the enzyme, and the activity and thermal tolerance were evaluated using the purified enzyme.

The enzyme concentration was obtained by mixing 10 µl of the diluted enzyme solution + 500 µl of Bradford solution (Quick Start^{™} Bradford 1x Dye Reagent, #5000205) and then measuring the absorbance at 595 nm.

The enzyme activity was measured by mixing 2.5 µl of 1 M citrate buffer at pH 5.5 with 50 µl of 1% xylan from beachwood (Megazyme, P-XYLNBE-10G), and then adding 47.5 µl of the diluted enzyme solution thereto, followed by carrying out the reaction at 50°C for 15 minutes. 150 µl of a DNS solution was mixed with the reaction solution to stop the reaction, and the mixture was boiled for 10 minutes to allow color development, and then cooled in ice water. The absorbance of the cooled reaction solution was measured at 540 nm, and the activity was measured using a standard curve obtained with xylose (Fluk Sigma-Aldrich, 95729).

In order to evaluate thermal tolerance, the purified enzyme was diluted to fall within the appropriate absorbance range, and incubated in a heat-block at 70°C for 5 minutes, and then the residual activity was measured.

**[Table 4]**

| Name of variant | Activity (U/mg) | Relative activity relative to Tr Xyn (%) | 70°C residual activity (%) |
|---|---|---|---|
| Tr Xyn (SEQ ID NO: 1) | 965.6 | 100 | 0 |
| Multiple variant (SEQ ID NO: 5) | 7205.3 | 622.2 | 85.6 |
| Multiple variant_T3I (SEQ ID NO: 17) | 8200.2 | 708.1 | 82.0 |
| Multiple variant_T3V (SEQ ID NO: 19) | 8942.9 | 772.3 | 104.1 |
| Multiple variant_R56K (SEQ ID NO: 21) | 6925 | 598 | 48.9 |
| Multiple variant_Y62F (SEQ ID NO: 23) | 6146.4 | 530.8 | 48.4 |
| Multiple variant_T146S (SEQ ID NO: 25) | 9556.5 | 825.3 | 65.7 |

As a result, when the enzyme activity was measured as a ratio, all 6 kinds of variants were confirmed to have improved thermal tolerance (residual activity) of 48.4% to 104.1% while having the enzyme activity in the range of 630% to 990% relative to the Tr Xyn (Table 4).

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A modified polypeptide having xylanase activity, wherein i) the modified polypeptide has a sequence identity of 70% or more and less than 100% with SEQ ID NO: 1; and/or
ii) the modified polypeptide is a polypeptide encoded by a polynucleotide having a sequence identity of 70% or more and less than 100% with a sequence encoding a mature polypeptide of SEQ ID NO: 1; and/or
iii) the modified polypeptide is a polypeptide encoded by (a) a mature polypeptide coding sequence of SEQ ID NO: 1, (b) a cDNA thereof, or (c) a polynucleotide that hybridizes to the full-length complement of (a) or (b) under low stringency conditions, medium stringency conditions, medium-high stringency conditions, high stringency conditions, or very high stringency conditions; and/or
iv) the modified polypeptide is a functional fragment of i), ii) or iii) polypeptide having xylanase activity; and
the modified polypeptide includes substitutions of one or more amino acids at positions 2, 3, 28, 38, 56, 62, 65, 80, 93, 114, 144, 146, 149, 151, 161, and 165;
wherein the position number is a position corresponding to the position of the polypeptide of SEQ ID NO: 1.

2. The modified polypeptide according to claim 1, wherein the modified polypeptide includes substitution of an amino acid at position 3.

3. The modified polypeptide according to claim 1, wherein the modified polypeptide includes modifications of amino acids at positions selected from below:
position 3;
position 56;
position 62;
position 146;
positions 3 + 56;
positions 3 + 62;
positions 3 + 146;
positions 56 + 62;
positions 56 + 146;
positions 62 + 146;
positions 3 + 56 + 62;
positions 3 + 56 + 146;
positions 3 + 62 + 146;
positions 56 + 62 + 146; and
positions 3 + 56 + 62 + 146;
wherein the position number is a position corresponding to the position of the polypeptide of SEQ ID NO: 1.

4. The modified polypeptide according to claim 1, wherein the modified polypeptide includes any one or more substitutions from i) to vi) below;
i) substitution of an amino acid at position 3 with valine or threonine;
ii) substitution of an amino acid at position 56 with arginine;
iii) substitution of an amino acid at position 62 with tyrosine; and
iv) substitution of an amino acid at position 146 with threonine;
wherein the position number is a position corresponding to the position of the polypeptide of SEQ ID NO: 1.

5. The modified polypeptide according to claim 1, wherein the modified polypeptide includes substitutions of 15 or more amino acids at positions 2, 3, 28, 38, 56, 62, 65, 80, 93, 114, 144, 146, 149, 151, 161, and 165.

6. The modified polypeptide according to claim 1, wherein the modified polypeptide includes any one or more substitutions selected from below:
13T/V ;
K56R ;
F62Y ;
S146T;
I3T/V + K56R ;
I3T/V + F62Y ;
I3T/V + S146T;
K56R + F62Y ;
K56R + S146T;
F62Y + S146T;
I3T/V + K56R + F62Y ;
I3T/V + K56R + S146T ;
I3T/V + F62Y + S146T;
K56R + F62Y + S146T;
I3T/V + K56R + F62Y + S146T ;
I3T/V + T2C + T28C + N38D + S65T + S80T + F93Y + V114T + H144K + S149T + N151T + Q161R + T165V;
K56R + T2C + T28C + N38D + S65T + S80T + F93Y + V114T + H144K + S149T + N151T + Q161R + T165V;
F62Y + T2C + T28C + N38D + S65T + S80T + F93Y + V114T + H144K + S149T + N151T + Q161R + T165V;
S146T + T2C + T28C + N38D + S65T + S80T + F93Y + V114T + H144K + S149T + N151T + Q161R + T165V;
13T/V + K56R + T2C + T28C + N38D + S65T + S80T + F93Y + V114T + H144K + S149T + N151T + Q161R + T165V;
13T/V + F62Y + T2C + T28C + N38D + S65T + S80T + F93Y + V114T + H144K + S149T + N151T + Q161R + T165V;
I3T/V + S146T + T2C + T28C + N38D + S65T + S80T + F93Y + V114T + H144K + S149T + N151T + Q161R + T165V;
K56R + F62Y + T2C + T28C + N38D + S65T + S80T + F93Y + V114T + H144K + S149T + N151T + Q161R + T165V;
K56R + S146T + T2C + T28C + N38D + S65T + S80T + F93Y + V114T + H144K + S149T + N151T + Q161R + T165V;
F62Y + S146T + T2C + T28C + N38D + S65T + S80T + F93Y + V114T + H144K + S149T + N151T + Q161R + T165V;
I3T/V + K56R + F62Y + T2C + T28C + N38D + S65T + S80T + F93Y + V114T + H144K + S149T + N151T + Q161R + T165V;
I3T/V + K56R + S146T + T2C + T28C + N38D + S65T + S80T + F93Y + V114T + H144K + S149T + N151T + Q161R + T165V;
I3T/V + F62Y + S146T + T2C + T28C + N38D + S65T + S80T + F93Y + V114T + H144K + S149T + N151T + Q161R + T165V;
K56R + F62Y + S146T + T2C + T28C + N38D + S65T + S80T + F93Y + V114T + H144K + S149T + N151T + Q161R + T165V; and
I3T/V + K56R + F62Y + S146T + T2C + T28C + N38D + S65T + S80T + F93Y + V114T + H144K + S149T + N151T + Q161R + T165V.

7. The modified polypeptide according to claim 1, wherein the modified polypeptide has increased thermal tolerance and/or thermal stability compared to a polypeptide composed of the amino acid sequence of SEQ ID NO: 1.

8. A method of producing xylo-oligosaccharide or xylose, the method comprising: contacting a xylan-containing material with the modified polypeptide of any one of claims 1 to 7, a host cell expressing the modified polypeptide, and/or a composition including the modified polypeptide.

9. A method of degrading xylan-containing materials, the method comprising: treating the modified polypeptide of any one of claims 1 to 7, a host cell expressing the modified polypeptide, and/or a composition including the modified polypeptide to a substrate.

10. A polynucleotide encoding the modified polypeptide of any one of claims 1 to 7.

11. A host cell comprising the modified polypeptide of any one of claims 1 to 7 and/or the polynucleotide of claim 10.

12. A method of preparing a modified polypeptide having xylanase activity, the method comprising the step of culturing the host cell of claim 11.
